# EUROPEAN PATENT APPLICATION

(11) **EP 3 626 830 A1**
(43) Date of publication of application: **25.03.2020**
(21) Application number: 18801295.9
(22) Date of filing: 15.01.2018
(51) Int. Cl.: C12Q 1/68

(54) **APOE PROMOTER SINGLE NUCLEOTIDE POLYMORPHISM ASSOCIATED WITH ALZHEIMER'S DISEASE RISK AND USE THEREOF**

(30) Priority: 15.05.2017 KR 20170060225; 17.05.2017 WO PCT/KR2017/005137
(71) Applicant: Infomeditech Co., Ltd., Seoul 06236 (KR)
(72) Inventor: LEE, Kun Ho, Gwangju 61746 (KR); CHOI, Kyu Yeong, Gwangju 62258 (KR); LEE, Jang Jae, Gwangju 61708 (KR)
(74) Representative: dompatent von Kreisler Selting Werner - Partnerschaft von Patent- und Rechtsanwälten mbB
(86) International application number: PCT/KR2018/000649
(87) International publication number: WO 2018/212427

(57) **Abstract**

The present invention relates to single nucleotide polymorphism (SNP) which can be used for Alzheimer's disease risk prediction. The SNP is rs405509 and is located on chromosome 19, 44905579 on the basis of human genome map GRCh38.p7. The present invention suggests that T allele of rs405509 plays the role of further increasing the risk of dementia of APOE E4/E4. The result of cerebral cortical thickness and hippocampal volume comparison between APOE genotypes suggests that the contraction of E4/E4 is greater than E3/E3 in Asians. The cerebral cortex of people having rs405509 T/T genotype among Caucasians having E4/E4 genotype is more severely atrophied. Therefore, the present invention enables confirmation of rs405509 T/T polymorphism along with APOE E4/E4 and thus can be used for diagnosing or risk-predicting Alzheimer's disease and/or dementia due to Alzheimer's disease.

<110>	Industry-Academic Cooperation Foundation, Chosun University

<120>	APOE promoter SNP associated with the risk of Alzheimer's disease
	and the use thereof

<130>	CSU18P-0001-WO

<150>	KR 10-2017-0060225
<151>	2017-05-15

<150>	PCT/KR 2017/005137
<151>	2017-05-17

<160>	6

<170>	KoPatentIn 3.0

<210>	1
<211>	28
<212>	DNA
<213>	Artificial Sequence

<220>
<223>	Forward primer - APOE promoter


<400>	1
ggggtaccga aagcagcgga tccttgat                                          28


<210>	2
<211>	28
<212>	DNA
<213>	Artificial Sequence

<220>
<223>	Reverse primer - APOE promoter


<400>	2
cccctcgagc ttcctgcctg tgattggc                                          28


<210>	3
<211>	28
<212>	DNA
<213>	Artificial Sequence

<220>
<223>	Forward primer - APOE rs405509 allele replacement


<400>	3
gaggagggtg tctggattac tgggcgag                                          28


<210>	4
<211>	28
<212>	DNA
<213>	Artificial Sequence

<220>
<223>	Reverse primer - APOE rs405509 allele replacement

<400>    4
ctcgcccagt aatccagaca ccctcctc                                          28


<210>    5
<211>    29
<212>    DNA
<213>    Artificial Sequence

<220>
<223>    Forward primer - APOE rs405509 allele replacement


<400>    5
gaggagggtg tctgtattac tgggcgagg                                         29


<210>    6
<211>    29
<212>    DNA
<213>    Artificial Sequence

<220>
<223>    Reverse primer - APOE rs405509 allele replacement


<400>    6
cctcgcccag taatacagac accctcctc                                         29

## Description

### Technical Field

The present invention relates to single nucleotide polymorphism for early diagnosis of Alzheimer's disease and for predicting risk for Alzheimer's disease. In addition, the present invention relates to methods for early diagnosis of Alzheimer's disease and for predicting risk for Alzheimer's disease, using a single nucleotide polymorphism. The present invention is based on a result obtained by performing the task of investigating Alzheimer's dementia-specific brain damage and constructing brain map based on MRI, which has been supported by the Brain Science Source Technology Development project of the Ministry of Science, ICT and Future Planning in Korea.

### Background Art

Alzheimer's disease is a progressive neurodegenerative disease characterized by cognitive decline and senile plaques in the brain. Alzheimer's disease is the most common cause of dementia in the elderly, and accounts for 60% to 80% of dementia (Barnes and Yaffe, 2011). It is estimated that about 13% of elderly people aged 65 or older and about 45% of elderly people aged 85 or older are patients with Alzheimer's disease. Alzheimer's disease (AD) is a complex neurodegenerative disease which causes not only memory loss and other cognitive disorders but also structural changes in the brain (Ballard et al., 2011; Forero et al., 2006). AD patients exhibit increased amyloid plaques in the posterior cingulate cortex (PCC), the medial prefrontal cortex, and the hippocampus in the brain, and a decreased hippocampal volume. In addition, the patients show a decreased level of beta-amyloid and increased levels of tau and p-tau in CSF (Jack, 2012; Trojanowski et al., 2010).

Therapeutic agents for Alzheimer's dementia are under development, but no noteworthy therapeutic agent is present until now. Accordingly, there is a high need for early diagnosis and early prediction of Alzheimer's dementia. As a technique for diagnosing Alzheimer's dementia, a neuropsychological test, an MRI brain imaging test, clinical diagnosis by specialist's opinion, a pathological test through cerebrospinal fluid and florbetaben-based amyloid-PET, and the like are mentioned. Clinical diagnosis can diagnose onset of dementia or mild cognitive impairment, but there may be cases where it is difficult to distinguish those from other brain diseases. The clinical diagnosis is not suitable for the purpose of early diagnosis, since diagnosis is only possible after symptoms develop. A cerebrospinal fluid test is performed through quantitative analysis such as analysis of beta amyloid protein and tau protein, and thus is a reliable diagnostic measure for dementia. However, subjects are very resistant against this test due to invasive cerebrospinal fluid collection. The pathological test through amyloid-PET is reliable but expensive. In a case of MRI brain imaging, techniques for investigating brain damage associated with dementia such as cerebral cortical atrophy and hippocampal atrophy and for early diagnosis are under development, but it is currently known that diagnosis is not made at an early time point. In addition, development of diagnostic techniques for dementia through blood is actively underway. However, there is a limitation to scale of populations for experiment and accuracy, and thus verification of reliability is required for clinical use.

In genetic factors, amyloid precursor protein, presenilin-1 and presenilin-2 are primary factors for early onset of Alzheimer's disease due to family history (Blennow et al., 2006; Hardy and Selkoe, 2002). In APOE, e4 allele is the strongest risk factor for late-onset of sporadic Alzheimer's disease (Bu, 2009; Corder et al., 1993; Huang and Mucke, 2012). The APOE gene has three polymorphisms, e2, e3, and e4 at frequencies of 8.4%, 77.9%, and 13.7%, respectively, from a global point of view (Farrer et al., 1997). The frequency of e4 in Alzheimer's disease dramatically increases to about 40% (Farrer et al., 1997). APOE is located in the long arm of chromosome 19 (19 q13.2). APOE has three alleles, that is, e2, e3, and e4, which are formed due to changes at amino acid positions 112 (Cys/Arg) and 158 (Arg/Cys), and there are 6-genotype (E2/E2, E2/E3, E3/E3, E2/E4, E3/E4, E4/E4) polymorphisms due to combinations of the three alleles. Among these, APOE e4 is usually found in 50% or more of AD patients. However, APOE e4 is found in only less than 15% of cognitively normal controls (Ward et al., 2012). Previous studies have reported that APOE e4 can shorten time of onset of AD by 5 to 15 years (Corder et al., 1993; Gomez-Tortosa et al., 2007). In addition, effects of APOE e4 on cognitive decline have been reported. However, some studies have reported that APOE e4 decreases cognitive ability; on the other hand, other studies have reported that APOE e4 has no effect on decreased cognitive ability, and results of yet other studies have suggested that APOE e4 slowly decreases cognitive ability (Anstey and Christensen, 2000; Beaudreau et al., 2013; Caselli et al., 2009; Craft et al., 1998; Deary et al., 2002; Jorm et al., 2007; Kleiman et al., 2006; Mayeux et al., 2001; Van Gerven et al., 2012). Despite such results which require discussion, healthy individuals with APOE e4 homozygotes exhibited a decreased hippocampal volume, whereas healthy individuals with APOE e4 heterozygotes showed no difference from non-e4-carrying subjects in the healthy elderly group (Crivello et al., 2010; Farlow et al., 2004; Lemaitre et al., 2005). In addition, APOE e4 was shown to be involved in conversion of mild cognitive impairment to Alzheimer's disease in the healthy elderly (Wang et al., 2011). Risk of e4 allele has been reported to vary among ethnic groups (Brainerd et al., 2013; Farrer et al., 1997; Heun et al., 2010; Hsiung and Sadovnick, 2007; Hsiung et al., 2004; Rose, 2005). However, even in a case where prediction is made based on APOE e4, explanations can be made only within 20% of degree of genetic impact on dementia. As such, e4-mediated risk and possible inducers for Alzheimer's disease still remain unclear. On the other hand, Korean Patent No. 10-1335021 describes association between patients with Alzheimer's disease or mild cognitive impairment and T/G heterozygosity of APOE rs405509.

Accordingly, the present inventors not only have studied APOE E4 genetic variation, but also have studied genetic variations, which may affect risk of APOE E4 for dementia, around the APOE gene. In addition, in a case of APOE E4/E4 homozygotes, the present inventors have identified that there are ethnic differences in risk for dementia, and have intended to analyze the cause. As a result, surprisingly, the present inventors have identified that a single nucleotide polymorphism located in the APOE promoter not only explains ethnic differences in risk for dementia of the APOE E4/E4 homozygotes, but also cerebral cortical thickness varies depending on the alleles. In addition, the present inventors have identified an effect of APOE polymorphism on a brain structure by analyzing cerebral cortical thickness and hippocampal volume.

### Disclosure of Invention

### Technical Problem

An object of the present invention is to provide a single nucleotide polymorphism (SNP) genetic variation for predicting Alzheimer's disease and/or risk for dementia caused by Alzheimer's disease.

Another object of the present invention is to provide a method for predicting Alzheimer's disease and/or risk for dementia caused by Alzheimer's disease by detecting a single nucleotide polymorphism (SNP) genetic variation.

### Solution to Problem

The present invention provides a single nucleotide polymorphism (SNP) that can be used to predict Alzheimer's disease and/or risk for dementia caused by Alzheimer. More specifically, the SNP is rs405509 which is located on chromosome 19 at 44905579, based on GRCh38.p7 version of the human genome map, and alleles thereof are T and G. The SNP showed ethnic differences in minor allele frequency (MAF).

In an embodiment of the present invention, the present invention predicts risk for dementia by isolating and purifying genomic DNA from a sample isolated from a subject, for example, cells, tissue, blood, or body fluid, and analyzing and identifying the APOE promoter rs405509 single nucleotide polymorphism and the APOE genotype.

In another embodiment of the present invention, there is provided a method for providing information about prediction of Alzheimer's dementia using APOE epsilon genetic variations (rs429358 and rs7412) and rs405509 genetic variation.

In yet another embodiment of the present invention, there is provided a method for diagnosing or predicting risk for mild cognitive impairment or Alzheimer's disease (AD) by detecting genetic variation indicative of mild cognitive impairment or Alzheimer's disease in a sample containing a nucleic acid which is isolated from a subject, the method comprising (a) a step of contacting the sample with a reagent capable of detecting the presence or absence of the genetic variation in a gene selected from genes encoding APOE E4 allele or gene products thereof; and (b) a step of contacting the sample with a reagent capable of detecting the presence or absence of the genetic variation selected from genes encoding rs405509 T allele of the APOE promoter or gene products thereof, wherein the presence of APOE E4/E4 and rs405509 T/T genetic variations indicates that the subject is a patient with mild cognitive impairment or Alzheimer's disease, or has high risk for mild cognitive impairment or Alzheimer's disease. Here, the method for predicting the risk may be performed by amplifying the nucleic acid isolated from the subject, and the nucleic acid may be genomic DNA or RNA.

In still yet another embodiment of the present invention, the method further comprises a step of obtaining an image, for example, a magnetic resonance (MR) brain image, for cerebral cortical thickness in the subject, wherein atrophy in cerebral cortical thickness may indicate that the subject is a patient with mild cognitive impairment or Alzheimer's disease, or has high risk for mild cognitive impairment or Alzheimer's disease.

In still yet another embodiment of the present invention, the method may further comprise a step of performing one or more of tests consisting of a neuropsychological test, a cerebrospinal fluid (CSF) test, and an amyloid-PET test.

In an embodiment of the present invention, the method detecting the presence or absence of the genetic variations is a method in which a nucleic acid in a single-stranded form obtained from the sample isolated from the subject is subject to form a complex with a complementary primer set, and then the presence or absence of the genetic variations is analyzed in the complex. The presence or absence of the genetic variations in the complex may be analyzed using polymerase chain reaction, nuclease digestion, hybridization, Southern blotting, restriction enzyme fragment polymorphism, sequencing, primer extension, or single-stranded conformation polymorphism, or using combinations thereof.

In still yet another embodiment of the present invention, there is provided a kit for diagnosing or predicting mild cognitive impairment or Alzheimer's disease, the kit capable of detecting the presence or absence of the genetic variation in a gene selected from genes encoding APOE E4 allele or gene products thereof, in which the kit may further include genes encoding the rs405509 T allele of the APOE promoter or gene products thereof. Thus, in an embodiment of the present invention, the kit may be a kit that includes primer sets which are complementary to single nucleotide polymorphism APOE E4 of APOE and APOE promoter single nucleotide polymorphisms rs405509 T/T, T/G, and GG, and a reaction enzyme.

The gene product in the present invention is a product resulting from expression of a gene and is understood to include RNA, for example, tRNA, mRNA, rRNA, miRNA and siRNA, a polypeptide, and a protein.

In another embodiment of the present invention, the kit may be a kit which includes an analytic method for analyzing genetic variation, a method for amplifying a nucleic acid, or a reference control.

In yet another embodiment of the present invention, there are provided complementary primer sets capable of detecting the presence or absence of the genetic variation in a gene selected from genes encoding APOE E4 allele or gene products thereof, and genes encoding the rs405509 T allele or gene products thereof may be provided together. Thus, in an embodiment of the invention, the primer sets may be primer sets which are complementary to single nucleotide polymorphism APOE E4 of APOE and APOE promoter single nucleotide polymorphisms rs405509 T/T, T/G, and GG.

In another embodiment of the present invention, the primer set may be a primer set to which a label, for example, a radioisotope, a fluorophore, or a chemical derivative is attached.

### Advantageous Effects of Invention

According to the present invention, risk for Alzheimer's dementia can be more accurately diagnosed by investigating genetic variation rs405509, in addition to APOE genotype, in particular, APOE E4/E4, as predictive genetic variation for sporadic Alzheimer's dementia.

### Brief Description of Drawings

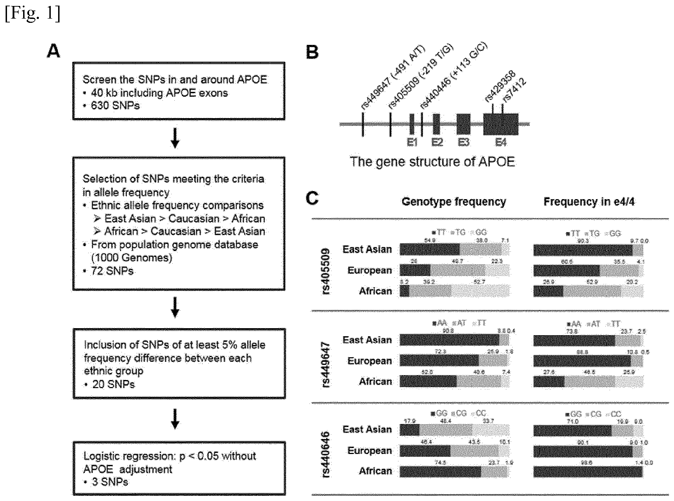

FIG. 1 illustrates a strategy for screening polymorphic candidates that regulate risk of APOE e4/4 (FIG. 1A). FIG. 1B schematically illustrates a gene structure of APOE. SNPs of rs449647 (-491 A/T), rs405509 (-219 T/G), rs440446 (+113 G/C), rs429358, and rs7412 were shown in panel B. FIG. 1C illustrates results obtained by evaluating distribution of genotype frequency for rs405509, rs449647, rs440446, regarding whole case-regulated subjects or e4 homozygotes from East Asian, Caucasian, and African ancestry. Here, the genotype frequency was calculated as an average frequency in each case and a control.

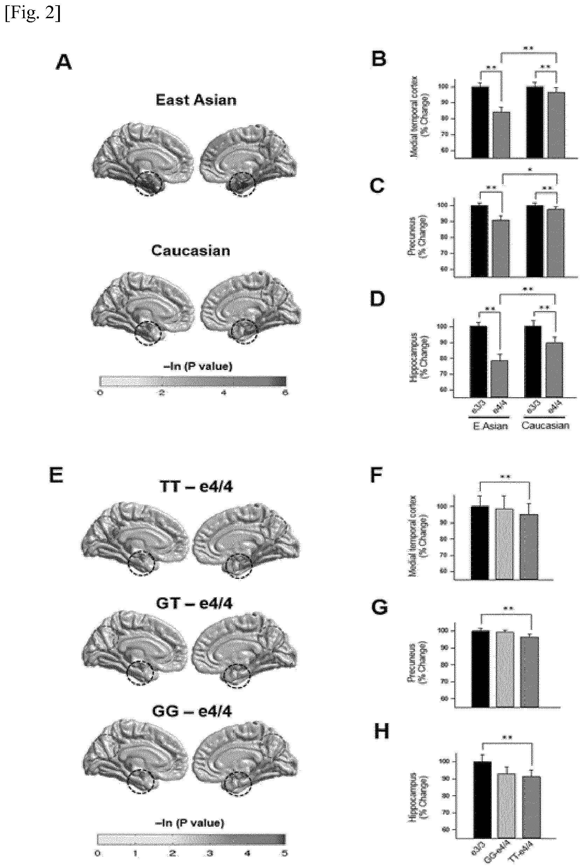

FIG. 2 illustrates a map showing decreased cortical thickness in East Asians and Caucasians (FIG. 2A). A point-wise difference in cortical thickness was estimated by applying a general linear model, and using APOE e4/4 and e3/3 as stationary factors, and age, sex, and field strength as covariates. The number represents a statistical difference (p < 0.05). The black circle represents brain atrophy in the medial olfactory and parahippocampal regions, and the red circle represents brain atrophy in the precuneus region (FIG. 2A). Average cortical thicknesses in the medial temporal cortex (medial olfactory and parahippocampal regions) (FIG. 2B) and in the precuneus (FIG. 2C) were compared depending on APOE genotypes in East Asians and Caucasians. Data were normalized to e3/3 and expressed as % with error bars having a 95% confidence interval. The bar graph is a result obtained by comparing hippocampal volumes between the APOE genotypes in East Asians and Caucasians (FIG. 2D).

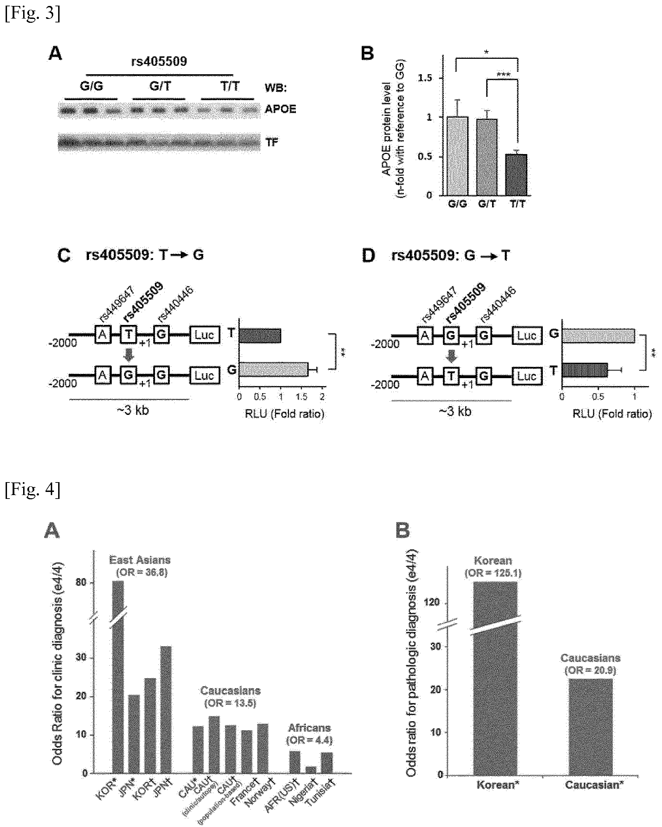

FIG. 3 illustrates that rs405509 T-allele decreases expression of the APOE protein. Human serum is subjected to Western blotting, together with anti-APOE and anti-transferrin (TF), to examine rs405509-dependent serum expression of the APOE protein (FIG. 3A). Serum samples are selected for rs405509 (G/G, G/T, and T/T) genotypes in APOE e3/3 homozygotes. The bar graph shows relative intensity of APOE expression (FIG. 3B). The GG genotype was used as a reference. Transferrin (TF) was used as a normalized control. Data are mean ± SEM (*p < 0.05, ***p < 0.001).
FIG. 4 illustrates susceptibility in Asians to onset of APOE e4/4-mediated Alzheimer's disease. For Alzheimer's disease, an odds ratio of APOE e4/4 with reference to e3/3 was compared among different ethnic groups. The bar graphs represent odds ratios of e4/4 for Alzheimer's disease based on clinical diagnosis (FIG. 4A) and for neuropathologically identified series (FIG. 4B). * Data set was analyzed by logistic regression, and † is a citation of the previous research result.

### Best Mode for Carrying out the Invention

In the present invention, the present inventors have analyzed, through a large scale study, susceptibility to APOE e4-mediated AD among different ethnic groups. As a result, e4 homozygotes were more vulnerable to Alzheimer's disease in East Asians as compared with Caucasian and African ancestry. In addition, the present inventors have identified such ethnic differences by an association study on patients diagnosed neuropathologically as Alzheimer's disease and controls. A small number of previous studies have reported different APOE e4 allele distributions in various populations (Farrer et al., 1997; Singh et al., 2006). The frequency of e4 allele is higher in the order of Africa, Europe, and Asia, which is the opposite of the order of risk of e4 for Alzheimer's disease.

It has been reported that APOE e4 promotes decreased cortical thickness in the entorhinal cortex, the parahippocampal cortex, and the precuneus (Donix et al., 2010; Foley et al., 2016; Thompson et al., 2011). Another study on cognitively normal subjects suggested that individuals with e4 also have decreased cortical thickness (Fouquet et al., 2014). In addition, individuals with e4 allele exhibited severe atrophy in the hippocampus which plays an important role in memory function, and memory impairment (Alexopoulos et al., 2011). In the present invention, the present inventors have found that APOE e4 homozygotes are associated with severely decreased cortical thickness in the entorhinal cortex, parahippocampal cortex, and precuneus regions, and hippocampal atrophy, in both East Asians and Caucasians, as compared with APOE e3 homozygotes, and this result was consistent with previous studies (Donix et al., 2010; Foley et al., 2016; Thompson et al., 2011). Surprisingly, it has been identified that these cortical and hippocampal atrophies observed in e4/4 subjects are more severe in East Asians than in Caucasians. The present invention suggests that East Asians with APOE e4/4 have increased risk for Alzheimer's disease as well as decreased cortical thickness and hippocampal atrophy in the brain, as compared with Caucasians.

The present inventors have hypothesized that differences in risk for e4/4-mediated AD among populations are due to ethnic differences in genetic background, in particular, polymorphisms around the APOE gene. APOE e4 is not the only APOE polymorphism associated with Alzheimer's disease. It has been reported that polymorphisms within the APOE promoter and intron regions regulate an effect of APOE e4 on onset of AD (Bertram et al., 2007; Lambert et al., 2002; Lambert et al., 1998b; Lescai et al., 2011). Two SNPs (rs449647 and rs405509) in the promoter and one SNP (rs440446) in the introns were evaluated to regulate an effect of APOE epsilon variations on Alzheimer's disease. It has been reported that -491AA and 219TT genotypes increase risk for AD independently of APOE epsilon genotypes (Lambert et al., 2002; Lambert et al., 1998a; Lambert et al., 1998b; Limon-Sztencel et al., 2016; Wang et al., 2000). In addition, it has been reported that rs405509 has a synergistic action with APOE e4 in influencing cognitive ability (Ma et al., 2016). The present inventors have analyzed that these SNPs exhibit different allelic frequencies in different ethnic populations. The present inventors have identified that in a case of rs405509 among these SNPs, East Asians including Koreans and Japanese carry a higher frequency of rs405509-T, which is a risk allele, in both the total population and the e4 homozygote-carrying group, as compared with Caucasian and African ancestry. This supports that e4 homozygotes have a high risk for Alzheimer's disease in East Asians as compared with other ethnic groups. Likewise, it has been identified that Caucasians have a high allelic frequency for rs405509-T as compared with African ancestry, and Caucasians also have a high e4/4 homozygote odds ratio as compared with African ancestry.

Several neuroimaging studies have reported an effect of APOE e4 homozygotes on brain atrophy; however, an effect of APOE promoter polymorphisms has not yet been clearly elucidated. Previous studies have reported an effect of rs405509 polymorphism in the APOE promoter region on the human brain in a non-dementia-related aging process in a Chinese population. It has been found that individuals having rs405509-TT along with APOE e4 exhibit atrophy in cortical thickness in an age-dependent manner, as compared with individuals having rs405509 G-allele (Chen et al., 2015a). The present inventors have found that individuals with rs405509-TT genotype are highly associated with Alzheimer's disease and cause strong atrophy in cortical thickness and hippocampal volume as compared with individuals having G-allele among e4 homozygotes. In particular, this pattern of decreased cortical thickness was observed in the medial temporal cortex (entorhinal and parahippocampal regions) and the precuneus, and atrophy in the parahippocampal region is similar to the previous study (Chen et al., 2015a). The present inventors have identified severely decreased cortical thickness in the entorhinal cortex, parahippocampal cortex, and precuneus regions, and hippocampal atrophy, in individuals having APOE e4 homozygote and rs405509-TT. In the present invention, the present inventors have identified that ethnic differences in Alzheimer's disease and risk for e4/4-mediated brain atrophy are due to ethnic diversity of rs405509 polymorphism.

In addition, the present inventors have demonstrated that rs405509-TT induces decreased expression of APOE in human brain and serum. In a reporter gene assay using allelic substitution at rs405509 T or G allele, the present inventors have demonstrated that an APOE gene having rs405509-T allele in the promoter is less expressed, suggesting that risk of containing rs405509-T is due to decreased APOE protein.

As described above, the present inventors have identified that APOE e4 homozygotes in East Asians are more vulnerable to Alzheimer's disease than Caucasian and African ancestry. Different allelic frequencies in APOE promoter polymorphisms among ethnic groups will make individuals having APOE e4 differentially susceptible to onset of Alzheimer's disease. In view of relevance of rs405509 to regulation of a level of the APOE protein, it is suggested that a decreased APOE level in e4 APOE individuals having rs405509-TT induces increased risk for Alzheimer's disease.

### Mode for the Invention

Hereinafter, the present invention will be described by way of examples. The following examples are for illustrative purposes only and the scope of the present invention is not limited thereto.

### Materials and methods

### (1) Samples and data

In the present invention, full-length genome information, MRI brain image information, and clinical diagnosis information were utilized. For 2,075 study subjects in the dementia cohort (from mainly Chosun University Hospital and other cooperative hospitals; average age of normal subjects: 73.45 ± 5.30 (mean ± SD), average age of patients with Alzheimer's disease: 71.65 ± 5.65 (mean ± SD), proportion of females in normal subjects: 62.4%, proportion of females in patients with Alzheimer's disease: 61.5%), full-length genomic information was acquired using the blood, and brain image information was acquired through MRI scan (3T MRI, Skyra, Siemens) of each study subject. Each study subject underwent neuropsychological tests (K-MMSE and Seoul Neuropsychological Screening Battery (SNSB)) necessary for diagnosis of dementia. As a result, diagnosis of normal, mild cognitive impairment, or dementia was made by a dementia clinic specialist. The result of diagnosis showed that 1,145 subjects were normal and 930 subjects were patients with dementia. In addition, a database of the National Research Center for Dementia (NRCD) in Gwangju, Korea, was used. All experimental and study protocols of the present invention were approved by the Board of Directors of Chosun University Hospital. All volunteers and relatives of patients submitted written consents which had been prepared prior to participating in the experiment. East Asian subjects consisted of 2,309 cognitively normal individuals and 1,886 AD patients. Genomes of Caucasians were acquired from the Alzheimer's Disease Neuroimaging Initiative (ADNI; http://adni.loni.usc.edu/) database, and brain images of the Caucasians were also obtained from the ADNI database. The Caucasians consisted of 515 normal subjects and 320 AD patients.

Clinical diagnosis of AD was made according to the AD evaluation criteria of the National Institute of Neurological and Communication Disorders and Stroke/Alzheimer's Disease and Related Disorders Association (NINCDS-ADRDA) (McKhann et al., 1984b). Mild cognitive impairment (MCI) was diagnosed according to currently agreed criteria (Winblad et al., 2004). The cognitively normal group is a group which has no neurological disease, and has no impairment of cognitive function or has no problems in daily life. Subjects with local lesion, head trauma history, or mental illnesses that may affect their mental ability, on brain MRI, were excluded. Subjects with mild medical abnormalities (for example, essential hypertension, diabetes without severe complications, or mild hearing impairment) were included.

For Caucasian genomes used for risk (odds ratio) of rs405509, data sets from the National Institute of Aging-Late Onset Alzheimer's Disease (NIA-LOAD), the National Cell Repository for Alzheimer's Disease (NCRAD), and the Alzheimer's Disease Neuroimaging Initiative (ADNI) were used. Two data sets or three data sets were combined and analyzed. An APOE promoter single nucleotide in individuals with E4/E4 genotype was used. In a case where the NIA-LOAD, the NCRAD, and the ADNI are combined and analyzed, individuals with E4/E4 genotype were 395 in total, among which 330 were patients with Alzheimer's dementia and 65 were normal subjects.

### (2) Brain amyloid imaging

¹⁸F-Florbetaben PET imaging was performed according to a conventional method (Osama Sabri, 2015, ClinTrans1 Imaging, Barthel, 2011, Lancet_neurology). Amyloid pathology was determined by brain amyloid-β plaque load (BAPL) scores. Florbetaben (¹⁸F) PET images for four brain regions (frontal cortex, posterior cingulate, lateral temporal cortex, and parietal cortex) were evaluated according to the set regional cortical tracer uptake (RCTU) scoring system (1 = no uptake, 2 = minor uptake, 3 = pronounced uptake). Next, the RCTU scores for the frontal cortex, the posterior cortex, the lateral temporal cortex, and the parietal cortex were summarized by brain amyloid-β plaque load (BAPL) scores (1 = no amyloid load, 2 = minor amyloid load, and 3 = significant amyloid load) in a single preset 3-grade scoring system for each PET scan. *In vivo* amyloid imaging was performed with positron emission tomography (PET) using Pittsburgh compound B (PiB). PiB deposition to a region of interest (ROI) in the brain was determined using the FreeSurfer version 5.1 software (Martinos Center for Biomedical Imaging), and a standardized uptake value ratio (SUVR) corrected for partial volume effects was calculated for each region of interest. The mean cerebral cortical SUVRs in the frontal cortex, posterior cortex, lateral temporal cortex, and parietal cortex regions were calculated by FreeSurfer. The cerebellar cortex was used as a region for reference. PiB-positive was defined as SUVR of 1.42, corresponding to the mean cerebral cortical binding potential of 0.18 which had been previously defined for PiB-positive. Caucasians' amyloid-PET data for both ¹⁸F-florbetapir and ¹¹C-PiB were obtained from the ADNI database (http://adni.loni.usc.edu).

### (3) Data generation and analysis

### SNP genotyping

Genomic DNA was extracted from peripheral blood leukocytes isolated from whole blood cells collected in EDTA tubes. Blood samples were centrifuged at 1500 × g for 10 minutes to remove plasma, and blood leukocytes were used for DNA extraction. The samples were genotyped using Affymetrix genome-wide genotyping arrays (Affymetrix® Axiom KORV1.0) optimized for Koreans. The KORV1.0 was designed by the Genome Science Center at the Korea National Institute of Health, and was obtained from the Korean Chip Consortium. In addition, genotyping analysis via Taqman or SNP type (Fluidigm) method was performed to evaluate accuracy in chip-based SNP genotyping. Genotyping data for the ADNI were obtained from the ADNI database (http://adni.loni.usc.edu).

### Genome-wide data imputation

Study data set from the NRCD consisted of 6,413 individuals. Data sets from the ADNI consisted of 818 individuals from the ADNI-1 data set, 432 individuals from the ADNI-GO/2 data set, and 818 individuals from the ADNI-WGS data set. Quality control for the samples included individual call-rate of < 95%, sex mismatch, heterozygosity (± 3SD from mean), redundancy or twins, SNPs with call-rate of <95%, SNPs with HWEP-value of < 10⁻⁶, and MAF of < 1%. Each data set was separately imputed using the HRC reference panel version 1.1. The HRC imputation from the NRCD was performed together with pre-phased study haplotypes by selecting East Asians as reference populations. For the ADNI data sets, imputation was separately performed together with pre-phased study haplotypes by selecting Europeans in the HRC Imputation Server as reference populations. After the imputation, SNPs (info < 0.5) which had been imputed at a low quality were excluded from further studies (McCarthy et al., 2016; Nagy et al., 2017; Surakka et al., 2016).

### (4) Imaging protocol

### MR image acquisition from Korean subjects

For 2,443 Korean subjects, a near 0.9 mm axial magnetization prepared rapid gradient-echo (MPRAGE) image in the entire brain was acquired with a 1.5 T MR scanner (Magnetom Avanto, Siemens) (TR = 1800 ms; TE = 3.43 ms; TI = 1100 ms; 15 flip angle; FoV = 224 × 224; matrix = 256 × 256; number of slices = 176). A near 0.8 mm sagittal MPRAGE volume was acquired with a 3 T MR scanner (Skyra, Siemens) (TR = 2300 ms; TE = 2.143 ms; TI = 900 ms; 9 flip angle; FoV = 256 × 256; matrix = 320 × 320; number of slices = 178.volumes). All images were acquired with an MR scanner at the Chosun University Hospital, Gwangju, Korea.

### MR images from ADNI

Baseline 1.5T and 3T MR images from 463 cognitively normal subjects, 796 subjects with mild cognitive impairment, and 310 AD patients were downloaded from the ADNI database (ADNI, http://adni.loni.use.edu/). All MR images for the subjects were acquired with 1.5T or 3T GE, Philips, and Siemens equipment according to the standard ADNI MPRAGE protocol. For a 1.5T scanner, nominal parameters for the MPRAGE protocol are as follows: sagittal plane, TR = 2300 to 2400 ms for multi-coil phased-array head coil (TR = 3000 ms for bird cage or volume head coil), minimum full TE, TI = 1000 ms, flip angle 8°, FOV = 240 × 240 mm, 192 × 192 in-plane matrix and 1.2 mm slice thickness. For a 3T scanner, nominal parameters for the MPRAGE protocol are as follows: sagittal plane, TR = 2300 or 3000 ms, minimum full TE, T1 = 853 to 900 ms, flip angle 8° to 9°, FOV = 256 to 260 × 240 mm, 256 × 256 in-plane matrix and 1.2 mm slice thickness (Jack et al., 2008). AD subjects were included based on scores from 20 to 26 on MMSE (Burns et al., 1998), and CDR = 0.5 or 1 (Morris, 1993). In addition, the selected AD group met the NINCDS/AARDA criteria which is criteria for probable AD. For healthy controls, individuals with CDR 0 and having no dementia were included (Wolz et al., 2011).

### (5) Image data processing and analysis

### MR image data processing

Image processing was performed with the public domain software FreeSurfer software (FreeSurfer 5.3.0) (FreeSurfer, http://surfer.nmr.mgh.harvard.edu/) used for brain structure analysis (Dale et al., 1999; Fischl and Dale, 2000; Fischl et al., 2004). Using DICOM-format MR images in an automated pipeline for FreeSurfer processing, a three-dimensional model for brain cortical surface was constructed (Dale et al., 1999; Dale and Sereno, 1993; Fischl et al., 1999a). In the processing, intensities of the images were normalized and the skull was removed from the normalized images. Next, subcortical segmentation processing was initiated with a connected components algorithm, and any holes found were filled to represent a single filled volume of the white matter for both cerebral cortical hemispheres. In addition, any metric distortions were decreased with tessellation and surface smoothening. Next, a refinement process was performed to create the gray/white matter boundary, and then initial surface model construction was performed. Next, the surface was deformed outward to form a pial surface. First, the shortest distance from each point on the gray/white matter surface to the pial surface was calculated, and the shortest distance from a point on the pial surface to the gray/white matter was calculated, so that thickness measurements of the brain cortex were obtained. Next, the final cortical thickness was obtained from the average of these two values at a particular point. Cortical thicknesses were measured for 163,842 vertices in each hemisphere with a triangular grid which is about 1 mm away from any point within the cortical mantle or another position representing a vertex. A cortical folding pattern was adjusted by smoothening the map with 0 mm, 5 mm, 10 mm, 15 mm, 20 mm, and 25 mm full-width-half-maximum Gaussian kernels and obtaining an average for the subjects with a non-rigid high-dimensional spherical averaging method. The adjusted map can detect even an mm or less difference among the subjects, and is not limited by voxel resolution. In addition, subcutaneous volume was acquired by an automated volume measurement method using FreeSurfer (Dale et al., 1999; Fischl and Dale, 2000; Fischl et al., 1999b).

### (6) Image statistical analysis

Differences in cortical thickness among the groups with different APOE alleles were statistically analyzed using the Surfstat (http://www.math.mcgill.ca/keith/surfstat/) toolbox for MATLAB (R2012a, The Mathworks, Natick, MA, USA). The present inventors statistically tested each unmasked point on the pial surface. A point-wise difference in cortical thickness was identified with a general linear model (GLM) using APOE alleles (e4/4 vs e3/3) as stationary factors, and sex, age, education level, and field strength as covariates (Fan et al., 2010). In order to evaluate rs405509 allelic differences, the subjects with haplotypes of T/TE4/4 and G/T-e4/4 + G/G-e4/4 were compared separately from the subjects with e3/3. In the GLM of rs405509, the present inventors identified a point-wise difference in cortical thickness by using APOE alleles (rs405509-e4/4 haplotype vs e3/3) as stationary factors, and sex, age, education level, and field strength as covariates (Chen et al., 2015b). In all cortical thickness analyses, random field theory (RFT)-based corrections for multiple point-wise cortical thickness comparisons were applied at a cluster level, and clusters that passed a false discovery rate (FDR) correction value of p < 0.05 were considered significant (Taylor and Adler, 2003) (http://www.math.mcgill.ca/keith/surfstat/). In addition, hippocampal volumes and anatomical regions of interest were statistically compared, using the R program (R version 3.3.1), for the groups carrying APOE alleles. Differences in hippocampal volume were evaluated with covariance analysis (ANCOVA) using APOE alleles (e4/4 vs e3/3, rs405509-e4/4 haplotype vs e3/3) as stationary factors, and sex, age, education level, diagnosis, and intracranial volume (ICV) as covariates (Hickie et al., 2005; Zierhut et al., 2013). Next, differences in region of interest (ROI) among different groups (e4/4 vs e3/3, rs405509-e4/4 haplotype vs e3/3) were compared using age, sex, education level, and diagnosis as covariates. In the analysis, a significance level was taken into account at p < 0.05; and if FDR correction was at a level of p < 0.05, the results were considered significant (Stricker et al., 2013; Ye et al., 2016).

### (7) Statistical analysis

Proportions of subjects in the control and the AD group were compared by chi-square tests. Odds ratios for events were calculated using a logistic regression analysis with a 95% confidence interval for both adjusted (sex and age) and unadjusted analyses (Basaria et al., 2010). All statistical tests were performed using both SPSS (version 23.0) and R program (version 3.3).

### (8) Expression of APOE gene from human blood sample

### Western blotting

First, serum was recovered from the whole blood (obtained from the NRCD). SDS-PAGE and Western blotting were performed according to the manufacturer's instructions with slight modifications (Amersham Biosciences, Piscataway, NJ). Membranes were blocked for 1 hour with 5% skim milk powder in PBS to which 0.1% Tween 20 was added, and subsequently incubated with primary antibodies for 1 hour at room temperature. The primary antibodies were diluted as follows and used: 1:1,000 rabbit anti-APOE (D719N; Cell Signaling); 1:1,000 rabbit anti-transferrin (ab109503; Abcam). Horseradish peroxidase (HRP)-conjugated secondary antibodies were used at 1: 5,000. Immunological activity was measured with the EZ-Western Lumi Plus (DoGen).

### (9) Reporter gene assay

The APOE gene (positions 1,983 to +935) promoter region was amplified, using the following primers, from genomic DNA from blood cells obtained from normal subjects and AD patients: Forward, 5'-GGGGTACCGAAAGCAGCGGATCCTTGAT3' (SEQ ID NO: 1); reverse, 5'-CCCCTCGAGCTTCCTGCCTGTGATTGGC3' (SEQ ID NO: 2). The amplified DNA was cleaved with KpnI and XhoI, and ligated into the pGL3.basic vector (Promega). The following primers were used to perform G → T and T → G allelic substitutions using a PCR-based site-directed mutagenesis of rs405509 (219G/T): T→G forward, 5'-GAGGAGGGTGTCTGGATTACTGGGCGAG-3' (SEQ ID NO: 3); reverse, 5'-CTCGCCCAGTAATCCAGACACCCTCCTC3' (SEQ ID NO: 4); G→T forward, 5'-GAGGAGGGTGTCTGTATTACTGGGCGAGG-3' (SEQ ID NO: 5); reverse, 5'-CCTCGCCCAGTAATACAGACACCCTCCTC-3' (SEQ ID NO: 6). The assay was performed using the PfuUltra High-Fidelity DNA Polymerase (Agilent).

### (10) Luciferase assay

HEK 293T cells were cultured in 12-well plates. After 24 hours, the cells were co-transfected with 0.25 µg of pGL3 having 0.25 µg of firefly luciferase reporter gene (Promega) and 0.25 µg of pCMV-β-galactosidase (Clontech) for 24 hours using the TransFectin (trade name) lipid reagent. The transfected cells were lysed with a reporter lysis buffer (Promega). Luciferase activity and β-galactosidase activity were quantified using the GloMax (trade name) luminometer (Promega) and the Epoch microplate spectrophotometer (BioTek), respectively. The luciferase activity was normalized to the β-galactosidase activity. The results represent three independent experiments.

### (11) Full-length genome information

Full-length genome information was obtained as follows: gDNA was extracted, with a QIAGEN DNA mini kit, using buffy coats isolated from the blood of normal subjects, patients with Alzheimer's mild cognitive impairment, and subjects who had been diagnosed as patients with Alzheimer's dementia, and then quantified. Quantification was performed using the ND-1000 spectrophotometer and Quant-iT PicoGreen dsDNA Reagent and Kits. Next, DNA quality was checked by performing electrophoresis on agarose 1.0% tris-borate-EDTA (TBE; Invitrogen) gels, and then microarray-based genotyping was performed for the samples that passed the DNA quality control. The Axiom Korean chip from Affymetrix was used according to the manufacturer's instructions to obtain information on each genotype. For SNPs which had not been microarrayed, genotyping was performed by an imputation method. Plink, EIGENSTRAT, Shapeit, impute2, GTOOL, EAGLETOOL, and minimac3 programs were used for full-length genome imputation, and the imputation was performed using, as reference panels, 1000 Genomes (https://www.ncbi.nlm.nih.gov/variation/tools/1000genomes/) and Michigan Imputation Server (https://imputationserver.sph.umich.edu/index.html).

### Results

### Association between APOE e4/4 and Alzheimer's disease

In terms of relevance to Alzheimer's disease, the present inventors analyzed risk of APOE variations in different ethnic groups of East Asian, Caucasian, and African ancestry. In all ethnic groups which include controls and patients diagnosed clinically as Alzheimer's disease, large differences in distribution of APOE genotypes and allele frequencies were exhibited between the controls and the group of patients with Alzheimer's disease. These results were more prominent in subjects with e4 of the group with Alzheimer's disease than in the controls. Although APOE e4 is an important risk factor for Alzheimer's disease, ethnic differences in e4-mediated risk have not been identified. In an analysis using patients, who had been clinically diagnosed as having a high likelihood of Alzheimer's disease, and normal controls, e4/4 subjects who are East Asians showed a very high odds ratio as compared with Caucasians (odds ratios: e4/4 = 33.40 and 15.86 for East Asians and Caucasians, respectively) (Table 1). In Table 1, the odds ratios were analyzed by logistic regression with reference to e3/3.

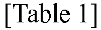

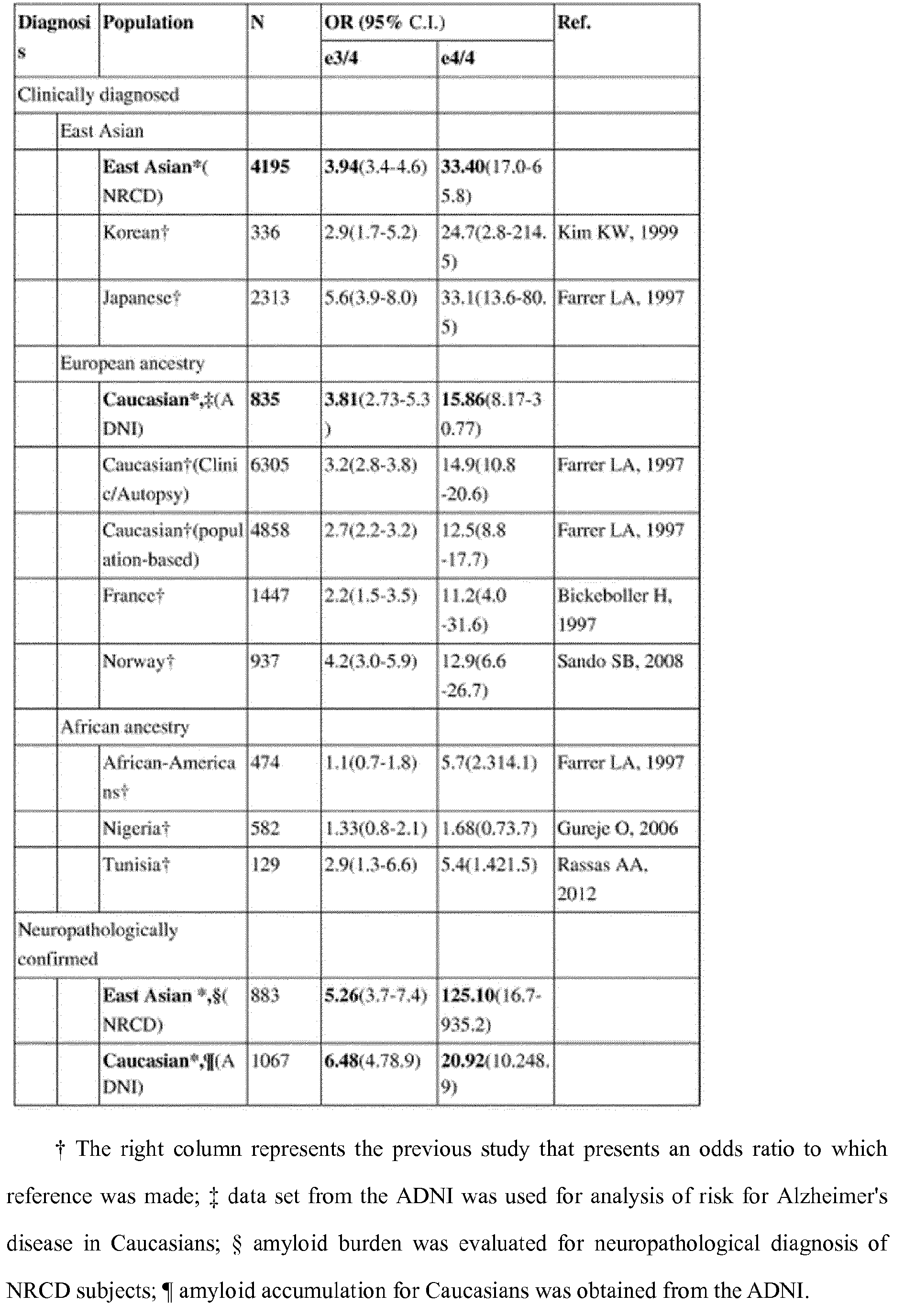

It was a very surprising result that e4/4-mediated risk is ethnic group-dependent and the risk was higher in the order of East Asians and Caucasians. The results of the present invention were consistent with previous studies that had analyzed relevance of the APOE genotype to Alzheimer's disease (Table 1). Table 2 below shows subjects in the Alzheimer's disease group and the control group based on clinical diagnosis.

**[Table 2]**

| Population | Total | Cognitively normal controls | | | | Alzheimer's disease cases | |
|---|---|---|---|---|---|---|---|
| | | n | Female (%) | Aget†(mean ± SD) | n | Female (%) | Age‡(mean ± SD) |
| East Asian§(NR CD) | 4195 | 2309 | 1423 (61.6%) | 74.6 ± 5.7 | 1886 | 1288 (68.3%) | 73.6 ± 5.6 |
| Caucasian( ADNI) | 835 | 515 | 265 (51.4%) | 74.38 ± 5.58 | 320 | 139 (43.4%) | 75.65 ± 6.84 |

| | | | | | | | |
|---|---|---|---|---|---|---|---|
| * The elderly aged equal to or older than 60 to less than 90; † the age in the control is an age at the time of examination; ‡ the age in the Alzheimer's disease group is an age at onset; § data set for East Asians was obtained from the NRCD; data set for Caucasians was obtained from the ADNI. | | | | | | | |

In order to identify ethnic differences in e4/4-mediated onset of Alzheimer's disease, the present inventors analyzed association therebetween using neuropathological diagnostic data sets from Koreans and Caucasians (Table 3).

**[Table 3]**

| Population | Tota1 | Aβ (-) † | | | Aβ (+) † | | |
|---|---|---|---|---|---|---|---|
| | | n | Female (%) | Age‡(mean ± SD) | n | Female (%) | Age§(mean ± SD) |
| East Asian (N RCD) | 883 | 557 | 321 (55.6) | 72.3 ± 6.3 | 306 | 162 (52.9) | 73.4 ± 6.0 |
| Caucasian(ADNI) | 1067 | 475 | 203 (42.7) | 72.8 ± 6.5 | 592 | 266 (44.9) | 74.3 ± 6.3 |

| | | | | | | | |
|---|---|---|---|---|---|---|---|
| * The elderly aged equal to or older than 60 to less than 90; † Ab (-) indicates amyloid-negative, Ab (+) indicates amyloid-positive; ‡ the age in the control group is an age at the time of examination; § the age in the Alzheimer's disease group is an age at onset; East Asians were Koreans who participated in amyloid-PET scanning; Caucasian data set for amyloid pathology was obtained from the ADNI. | | | | | | | |

In both ethnic groups, more e4 carriers were identified in the amyloid-positive group than in the amyloid-negative group (Table 4).

**[Table 4]**

| | APOE | n | Genotypes frequencies (%) | | | | | | Allele frequencies (%) | | |
|---|---|---|---|---|---|---|---|---|---|---|---|
| | Genotype | | e2/2 | e2/3 | c2/4 | e3/3 | e3/4 | c4/4 | c2 | c3 | c4 |
| East Asian(NR CD) | Aβ(-)^{∗} (%) | 57 7 | 0.3 | 10.4 | 0.5 | 74.3 | 14.2 | **0.2** | 5.8 | 86.6 | **7.6** |
| | Aβ(+)^{∗} (%) | 30 6 | 0.6 | 2.6 | 1.6 | 42.8 | 42.5 | **9.8** | 2.8 | 65.4 | **31.9** |
| | γ*2* (P) | | 180.60 (4.010⁻³⁷) | | | | | | 178.33 (1.810⁻³⁹) | | |
| Caucasian (ADNI) | Aβ (-) (%) | 47 5 | 0.4 | 13.7 | 1.1 | 66.1 | 17.1 | **1.7** | 11.5 | 73.5 | **15.0** |
| | Aβ (+) (%) | 59 2 | 0 | 2.5 | 2.9 | 33.4 | 47.4 | **13.2** | 3.5 | 54.8 | **41.7** |
| | γ2 (P) | | 203.05 (4.51x**10⁻⁴⁶)** | | | | | | 122.16 (2.1x10⁻²⁸) | | |

| | | | | | | | | | | | |
|---|---|---|---|---|---|---|---|---|---|---|---|
| * Ab(-) indicates an amyloid-negative subject, and Ab(+) indicates an amyloid-positive subject. | | | | | | | | | | | |

In a study for association between APOE genotype and amyloid accumulation, Korean e4/4 subjects showed a remarkably high odds ratio as compared with Caucasians (odds ratios for e4/4 = 125.1 and 20.92 for Koreans and Caucasians, respectively), which was consistent with results from the clinically diagnosed subjects (Tables 1 and 5). These results suggest that East Asian e4 homozygotes are more vulnerable to Alzheimer's disease than other ethnic groups.

**[Table 5]**

| Population | n | e3/3 † | e2/2 + e2/3 | | e2/4 | | e3/4 | | e4/4 | |
|---|---|---|---|---|---|---|---|---|---|---|
| | | | OR‡ (95% C.I.) | P | OR (95% C.I.) | P | OR (95% C.I.) | P | OR (95% C.I.) | P |
| East Asian(N RCD) | 883 | Ref | 0.49(0. 2-0.9) | 0.049 | 5.33(1.2 -22.8) | 0.024 | 5.26(3.7-7 .4) | 1.910⁻²¹ | 125.10(16.7-935. 2) | 3.010⁻⁶ |
| Caucasian(ADNI ) | 106 7 | Ref | 0.38(0. 2-0.7) | 0.001 5 | 6.27(2.4 -19.7) | 0.0005 | 6.46(4.7-8 .9) | 2.810⁻³⁰ | 20.92(10.2-48.9) | 1.310^{-1 4} |

| | | | | | | | | | | |
|---|---|---|---|---|---|---|---|---|---|---|
| * Association between APOE genotype and amyloid-based neuropathological diagnosis in different ethnic groups was analyzed by logistic regression; † e3/3 was used as a reference genotype; ‡ the odds ratio was adjusted according to sex and age. | | | | | | | | | | |

### Screening of polymorphism around APOE Gene

It was estimated that ethnically different e4/4 risk which is in the order of East Asian, Caucasian, and African ancestry is due to genetic polymorphisms around APOE. In order to examine the causative mechanism, the present inventors screened SNPs within a 40 kb region of a gene around APOE which are associated with allele frequencies among different ethnic groups (FIG. 1A). Of the 630 imputed SNPs spanning the 40 kb, 72 SNPs were selected according to criteria, that is, criteria such as an allelic frequency in the order of East Asian, Caucasian, and African ancestry or vice versa. Subsequently, the present inventors removed SNPs according to significant relevance to Alzheimer's disease and minor allelic frequencies without adjustment of APOE. As a result, three SNPs, rs449647, rs405509, and rs440446 were derived. These polymorphisms were located in the APOE promoter and intron regions (FIG. 1B). These showed large differences in allelic (rs449647-A, rs405509-T, and rs440446-G) frequencies among East Asian, Caucasian, and African ancestry (FIG. 1C). Among these, rs405509 showed remarkable differences among the ethnic groups (based on 1000 genome database, T-allele frequency = 0.67, 0.48, and 0.24 for East Asian, Caucasian, and African ancestry, respectively). In particular, e4 homozygotes from East Asians had TT-genotype of 90.4%, while those from Caucasians and Africans had TT-genotypes of 64.2% and 21.3%, respectively, indicating that a difference in allelic frequency of rs405509 may provide a difference in susceptibility to AD onset for e4/4 among different ethnic groups. rs449647 and rs440446 showed different allelic frequencies among different ethnic groups, but frequencies thereof in e4/4 were not largely different among the ethnic groups.

### Association between rs405509 and Alzheimer's disease

In order to examine whether TT genotype of rs405509 confers high risk of Alzheimer's disease in e3/4 and e4/4, the present inventors tested relevance of rs405509 in e3/4 and e4/4 subjects. As expected, rs405509-TT showed a remarkably high odds ratio in both the 3/4 and e4/4 subject groups from the NRCD data set (East Asians) (Table 6), suggesting an ethnic difference in risk for e4/4. E4 homozygotes with a high rs405509-TT frequency in East Asians exhibited higher susceptibility to Alzheimer's disease than Caucasian and African ancestry.

**[Table 6]**

| **Group** | | **Total(n)** | **APOE** e3/4(n) | **rs405509 in e3/4(TT** vs GT+GG) | | **APOE** e4/4(n) | **rs405509 in e4/4(TT** vs GT+GG) | |
|---|---|---|---|---|---|---|---|---|
| | | | | **OR†**(95% C.I.) | **P** | | **OR†**(95% C.I.) | **P** |
| NRC D | NP | 14322 | 2272 | 1.40(1.19-1.65) | 6.59x10⁻⁰⁵ | 140 | 3.59(1.94-6. 62) | 4.48 x 10⁻⁰⁵ |
| | AD | 2079 | 855 | | | 197 | | |

| | | | | | | | | |
|---|---|---|---|---|---|---|---|---|
| † The odds ratio of rs405509-TT genotype was analyzed by logistic regression in e3/4 and e4/4 genotype subjects with reference to rs405509-GT+GG genotypes; ‡ NP was a normal population control; § AD was a patient with Alzheimer's disease. | | | | | | | | |

In addition, in order to identify an effect of rs405509 on risk of e3/4- and e4/4-mediated Alzheimer's disease, the present inventors analyzed relevance of e3/4 and e4/4 in other rs405509 genotype backgrounds, that is, rs405509-TT, GT, and GG (Table 7). As a result, e3/4 and e4/4 subjects exhibited a higher odds ratio for the rs405509-TT genotype than the rs405509-G allele background in both NRCD and ADNI data sets.

**[Table 7]**

| **Datase t** | **rs405509** | **n** | **e3/3** | **e3/4** | | **e4/4** | |
|---|---|---|---|---|---|---|---|
| | | | | **Odds ratio** (95% C.I.) | **P** | **Odds ratio** (95% C.I.) | **P** |
| **NRCD** | TT | 8773 | ref | 4.23 (3.714.81) | 2.55E-105 | 15.53 (12.14 19.87) | 1.39E-105 |
| | GT | 7212 | ref | 3.69 (3.084.41) | 3.90E-46 | 7.50(3.62 15.57) | 6.24E-8 |
| | GG | 7183 | ref | 2.09 (1.034.23) | 0.041 | 4.36 (0.18 107.99) | N/A |
| **ADNI** | TT | 265 | ref | 6.79(3.6713.03) | 2.80E-9 | 22.83(7.89 85.42) | 1.54E-7 |
| | GT | 482 | ref | 5.03 (3.277.85) | 3.75E-13 | 11.85 (4.22 42.72) | 1.83E-5 |
| | GG | 197 | ref | 5.09 (2.0913.63) | 5.73E-4 | N/A | N/A |

| | | | | | | | |
|---|---|---|---|---|---|---|---|
| * Odds ratios of APOE genotypes e3/4 and e4/4 were analyzed by logistic regression in respective rs405509 genotype data sets (rs405509-TT, rs405509-GT, and rs405509-GG genotypes) with reference to e3/3 for Alzheimer's disease. | | | | | | | |

### Association between APOE e4/4 and brain atrophy

In order to identify whether ethnic differences are exhibited in brain atrophy mediated by APOE e4/4, the present inventors performed point-wise cortical thickness analysis, through a general linear model, between e4/4 and e3/3 subjects for East Asians and Caucasians. In both East Asians and Caucasians, e4/4 subjects exhibited a thinner cortical area than e3/3 (FIGS. 2A, 2B, and 2C). Areas where e4/4-dependent contraction occurred were the entorhinal-parahippocampal-fusiform, the precuneus, and the inferior parietal lobule (P < 0.05). Surprisingly, decreased cortical thickness in these areas was much strongly exhibited in East Asians as compared with Caucasians. Covariance analysis (ANCOVA) was performed to measure ethnic differences in e4/4-induced cortical atrophy. A relative mean cortical thickness in the areas where cortical atrophy had occurred was calculated for e4/4 subjects with reference to e3/3. In both East Asians and Caucasians, a large difference was exhibited between e4/4 and e3/3 in terms of mean cortical thicknesses within the medial temporal cortex including the entorhinal cortex and parahippocampal cortex, and the precuneus (*P < 0.05, ** P < 0.01). In addition, the East Asians exhibited a larger difference between e4/4/and e3/4 than in the Caucasians (FIGS. 2B and 2C). A direct comparison (normalized to e3/3) of relative cortical contraction in e4/4 between the East Asians and the Caucasians also showed significant results (*P < 0.05, **P < 0.01). In addition, the present inventors analyzed hippocampal volumes between e4/4 and e3/3 subjects in the East Asians and the Caucasians. In both East Asians and Caucasians, hippocampal atrophy was more remarkably exhibited in e4/4 than in e3/3, and an e4/4-induced hippocampal atrophy rate as compared with e3/3 was higher in the East Asians than in the Caucasians (**P < 0.01) (FIG. 2D). These results suggest that the East Asians are more vulnerable to APOE e4/4-mediated brain atrophy, including decreased cortical thickness and hippocampal contraction. In order to examine an effect of rs405509 on brain atrophy in e4/4 homozygotes, the present inventors analyzed decreased cortical thickness depending on e4/4 and respective genotype combinations of rs405509 (e4/4-TT, e4/4-GT, and e4/4-GG) with reference to e3/3 in Caucasian subjects (FIGS. 2E, 2F, and 2G). E4/4 subjects with rs405509-TT exhibited remarkable clusters within the medial temporal cortex (entorhinal cortex and parahippocampal cortex) and the precuneus (P < 0.05). On the other hand, e4/4 subjects with rs405509-G allele did not exhibit such clusters. Quantitative comparative analysis with ACNOVA showed that e4/4 subjects with r405509-TT genotype exhibit remarkable atrophy in the medial temporal cortex (F = 8.38, P < 0.01) and the precuneus (F = 10.707, P < 0.01) as compared with e3/3 subjects, whereas e4/4 subjects with rs405509-GG did not exhibit any significance in cortical area (FIGS. 2F and 2G). The present inventors also tested hippocampal atrophy in e4/4 subjects with rs405509-TT and rs405509-GG in Caucasians. Hippocampal contraction in e4/4 subjects with rs405509-TT was more strongly exhibited than in e3/3 subjects, whereas contraction in e4/4 subjects with rs405509-GG was not remarkable (FIG. 2H). These neuroimaging results support that TT genotype in rs405509 affects not only risk for Alzheimer's disease but also is associated with brain contraction in e4/4 subjects. Table 8 shows the subjects who had participated in neuroimaging analysis.

**[Table 8]**

| | East Asian*(NRCD) | Caucasiant(ADNI) |
|---|---|---|
| n | 1684 | 83 1 |
| Age§ (y, mean ± SD) | 72.77 ± 5.12 | 74.70 ± 6.59 |
| Female (n, %) | 1028 (61.0%) | 358 (43.0%) |
| Years of education (y, mean ± SD) | 9.01 ± 5.10 | 16.05 ± 2.78 |
| MMSE (mean ± SD) | 26.01 ± 3.84 | 27.43 ± 2.54 |

| | | |
|---|---|---|
| * East Asian means participants in the NRCD at Chosun University in Gwangju, Korea; † Caucasian means participants in the ADNI cohort; § the elderly were aged equal to or older than 60 to less than 90. | | |

### Expression of APOE

According to the present invention, rs405509 is associated with onset of Alzheimer's disease and brain contraction in e4 homozygotes. Therefore, the present inventors analyzed biological relevance of rs405509 to regulation of APOE. In order to examine whether rs405509 genotype regulates expression of the APOE protein, the present inventors performed Western blotting for human sera of rs405509-TT, GT, and GG genotypes in e3/3 subjects (FIG. 3A). A level of the APOE protein in the subjects with rs405509-TT was decreased to a surprising level as compared with the level in the subjects with rs405509-GG and GT (FIG. 3A). These results were consistent with previous studies, suggesting that a decreased level of APOE is associated with increased risk for Alzheimer's disease. In addition, an effect of rs405509 on transcriptional activity of APOE was identified through a reporter gene assay. The assay was performed using APOE promoter fragments from AD patients with rs405509-T allele and cognitively normal individuals with rs405509-G. The rs405509 allele in each promoter region was changed to an alternative-allele, and then a luciferase-based reporter gene assay was performed (FIGS. 3C and 3D). A single nucleotide polymorphism from T to G at the rs405509 position resulted in a surprising increase in APOE promoter activity (160% relative to the control), whereas substitution from G to T resulted in a dramatic decrease in the promoter activity (66% relative to the control). This suggests that the rs405509-T allele decreases APOE transcription as compared with the G allele. These results suggest that rs405509 regulates an effect of e4/4 by regulating a level of the APOE protein.

### Industrial Applicability

The present invention provides a method that enables a more accurate prediction for risk of dementia by analyzing, in genomic DNA collected from blood or a biological sample, APOE genotype as well as genetic variation of rs405509 in the APOE promoter. The present invention can contribute to future diagnosis techniques based on big data for dementia together with complex clinical and pathological information such as MRI brain image, neuropsychological test, cerebrospinal fluid (CSF) test, and amyloid-PET test.

## Claims

1. A method for diagnosing or predicting risk for mild cognitive impairment or Alzheimer's disease (AD) by detecting genetic variation indicative of mild cognitive impairment or Alzheimer's disease in a sample containing a nucleic acid which is isolated from a subject, the method comprising:
(a) a step of contacting the sample with a reagent capable of detecting the presence or absence of the genetic variation in a gene selected from genes encoding APOE E4 allele or gene products thereof; and
(b) a step of contacting the sample with a reagent capable of detecting the presence or absence of the genetic variation selected from genes encoding rs405509 T allele of the APOE promoter or gene products thereof,
wherein the presence of APOE E4/E4 and rs405509 T/T genetic variations indicates that the subject is a patient with mild cognitive impairment or Alzheimer's disease, or has risk for mild cognitive impairment or Alzheimer's disease.

2. The method of claim 1, wherein a method of detecting the presence or absence of the APOE E4/E4 and rs405509 T/T genetic variations includes obtaining a nucleic acid in a single-stranded form from the sample isolated from the subject, allowing the nucleic acid to form a complex with a complementary primer set, and then analyzing the presence or absence of the genetic variations in the complex.

3. The method of claim 2, wherein the analyzing the presence or absence of the APOE E4/E4 and rs405509 T/T genetic variations in the complex includes performing analysis using polymerase chain reaction, nuclease digestion, hybridization, Southern blotting, restriction enzyme fragment polymorphism, sequencing, primer extension, or single-stranded conformation polymorphism, or using combinations thereof.

4. The method of claim 1, wherein the nucleic acid obtained from the sample isolated from the subject is amplified.

5. The method of claim 1, wherein the nucleic acid obtained from the sample isolated from the subject is genomic DNA.

6. The method of claim 1, wherein the nucleic acid obtained from the sample isolated from the subject is RNA.

7. The method of claim 1, further comprising:
a step of obtaining an image for cerebral cortical thickness in the subject,
wherein atrophy in the cerebral cortical thickness indicates that the subject is a patient with mild cognitive impairment or Alzheimer's disease, or has risk for mild cognitive impairment or Alzheimer's disease.

8. The method of claim 7, wherein the image for cerebral cortical thickness is an MR brain image.

9. The method of any one of claims 1, 7, and 8, further comprising:
a step of performing one or more of tests consisting of a neuropsychological test, a cerebrospinal fluid (CSF) test, and an amyloid-PET test.

10. A kit for diagnosing or predicting mild cognitive impairment or Alzheimer's disease, in which the presence or absence of APOE E4/E4 genetic variation is detected in a gene selected from genes encoding APOE E4 allele or gene products thereof, and the presence or absence of rs405509 T/T genetic variation selected from genes encoding rs405509 T allele of the APOE promoter or gene products thereof is additionally detected.

11. The kit of claim 10, comprising:
primer sets which are complementary to the APOE E4 allele and the rs405509 T allele of the APOE promoter; and
a reaction enzyme.

12. The kit of claim 10, wherein analytic methods for analyzing the genetic variations are included.

13. The kit of claim 10, wherein a method for amplifying a nucleic acid is included.

14. The kit of claim 10, wherein a reference control is included.
